# FASCICULE DE BREVET EUROPEEN

(11) **EP 3 303 600 B1**
(45) Date de publication et mention de la délivrance du brevet: **27.08.2025**
(21) Numéro de dépôt: 16733149.5
(22) Date de dépôt: 26.05.2016
(51) Int. Cl.: C12P 7/10

(54) **PROPAGATION ET SACCHARIFICATION SIMULTANÉES DE LA LEVURE**
GLEICHZEITIGE VERMEHRUNG UND VERZUCKERUNG VON HEFE
SIMULTANEOUS YEAST PROPAGATION AND SACCHARIFICATION

(30) Priorité: 29.05.2015 FR 1554902
(43) Date de publication de la demande: 11.04.2018
(73) Titulaire: Lesaffre et Compagnie, 75001 Paris (FR); Agro Industrie Recherches et Developpements A.R.D., 51110 Pomacle (FR); Institut national de recherche pour l'agriculture, l'alimentation et l'environnement, 75007 Paris (FR)
(72) Inventeur: GHISONI, Flora, 06640 Saint Jeannet (FR)
(74) Mandataire: Plasseraud IP
(86) Numéro de dépôt international: PCT/FR2016/051237
(87) Numéro de publication internationale: WO 2016/193576

(56) Documents cités:
- WO-A1-2009/155633
- WO-A1-2014/072232
- HARNER NICOLE K ET AL: "Mutants of the pentose-fermenting yeast Pachysolen tannophilus tolerant to hardwood spent sulfite liquor and acetic acid", ANTONIE VAN LEEUWENHOEK, vol. 105, no. 1, January 2014 (2014-01-01), pages 29 - 43, XP002754253
- HARNER NICOLE K ET AL: "Genetic improvement of native xylose-fermenting yeasts for ethanol production", JOURNAL OF INDUSTRIAL MICROBIOLOGY & BIOTECHNOLOGY, vol. 42, no. 1, January 2015 (2015-01-01), pages 1 - 20, XP002754254
- CHO K M ET AL: "Novel SSF process for ethanol production from microcrystalline cellulose using the -integrated recombinant yeast, Saccharomyces cerevisiae L2612 GC", JOURNAL OF MICROBIOLOGY AND BIOTECHNOLOGY JUNE 1999 KOREAN SOCIETY FOR MICROBIOLOG AND BIOTECHNOLOGY KOR, vol. 9, no. 3, June 1999 (1999-06-01), pages 340 - 345, XP002754255, ISSN: 1017-7825
- GANTI S MURTHY ET AL: "A simultaneous saccharification and fermentation model for dynamic growth environments", BIOPROCESS AND BIOSYSTEMS ENGINEERING, SPRINGER, BERLIN, DE, vol. 35, no. 4, 11 October 2011 (2011-10-11), pages 519 - 534, XP035044353, ISSN: 1615-7605, DOI: 10.1007/S00449-011-0625-9
- NIELSEN F ET AL: "Short-term adaptation during propagation improves the performance of xylose-fermenting Saccharomyces cerevisiae in simultaneous saccharification and co-fermentation", BIOTECHNOLOGY FOR BIOFUELS 20151221 BIOMED CENTRAL LTD. GBR, vol. 8, no. 1, 21 December 2015 (2015-12-21), XP002754256, DOI: 10.1186/S13068-015-0399-4

## Description

La présente invention se rapporte au domaine des levures utilisées dans la fabrication de biocarburants et d'autres composés de chimie verte, produits par un processus de fermentation.

La diminution des stocks d'énergies fossiles a mené l'industrie à chercher des solutions alternatives, mettant en œuvre autant que possible des matières premières renouvelables et des processus moins polluants. On citera parmi ces solutions la fabrication de bioéthanol à partir de biomasse végétale, de biomasse issue de déchets végétaux ou même de déchets municipaux. Afin d'être acceptées, les versions vertes des composés chimiques doivent être aussi efficaces, voire plus, que les versions existantes, et leurs procédés de fabrication doivent être économiquement compétitifs.

Les applications sont nombreuses : les carburants de première génération, biodiesel ou éthanol, issus de matière première végétale comme la canne à sucre, la betterave, le blé, le maïs ou l'huile végétale, les carburants de deuxième génération, biodiesel, bio-kérosène, éthanol cellulosique, issus de biomasse végétale non alimentaire ou de résidus de culture, et d'autres produits de chimie lourde ou de chimie fine.

La matière première doit être prétraitée. Selon son origine, le pré-traitement est mécanique (râpage, broyage, hachage, moulin, pression), thermique, ou chimique. Le marc brut obtenu est soumis à une extraction et/ou à une hydrolyse enzymatique. Ceci conduit à un substrat fermentescible auquel est ajouté le microorganisme fermenteur. Enfin, le produit de fermentation peut être utilisé pour extraire des produits d'intérêt, par exemple par distillation ou extraction au moyen d'un solvant. Différentes étapes de ce processus constituent des verrous technologiques et ont été largement étudiés : prétraitement de la biomasse pour la rendre accessible aux enzymes, définition des mélanges enzymatiques pour une hydrolyse efficace des polymères de carbohydrates ou encore fermentation alcoolique des différents sucres obtenus (pentoses, hexoses).

L'étape de propagation alcoolique de la levure, objet de la présente invention, est peu décrite. A ce titre, la présente invention porte sur un procédé de propagation de levures, pour l'utilisation dans la production d'un produit de fermentation à partir de biomasse lignocellulosique, comprenant les étapes consistant à :
a) disposer d'un réacteur
b) mettre en contact dans ledit réacteur
   - une population de levures aptes à métaboliser les pentoses et les hexoses, à raison de 0,2 à 2,0 g de matière sèche de levures par kg de milieu complet préparé, où les levures de la population sont des levures d'une souche de levure choisie parmi les souches déposées à la CNCM sous les numéros suivants : I-4624, I-4625, I-4626, I-4627 et I-4783,
   - le marc brut issu du prétraitement de la biomasse lignocellulosique sans étapes d'hydrolyse ni de séparation à raison d'une teneur en matière sèche (MS) comprise entre 8 et 15%,
   - des nutriments,
   - des cellulases, à raison de 5 à 15 mg de protéine par gramme de MS,
c) incuber le mélange à une température comprise entre 25 et 38°C, de préférence entre 28 et 33°C, et en micro-aérobiose,
dans lequel la saccharification du marc brut et la croissance des levures sont réalisées simultanément.

### Brève description des figures :

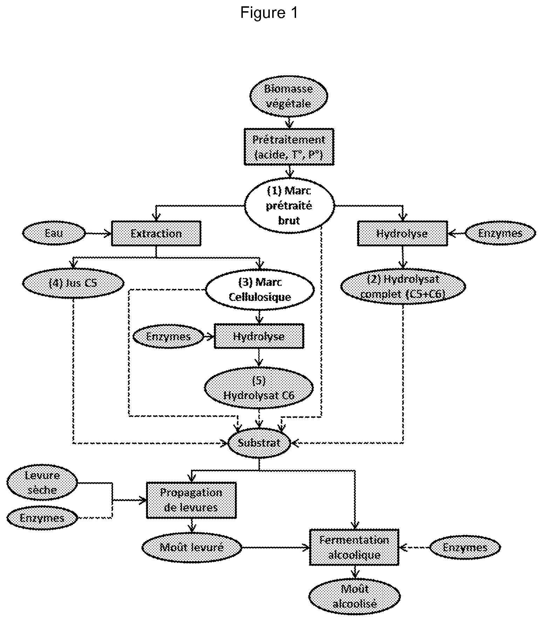

La Figure 1 présente un procédé de production de bio-éthanol comprenant le prétraitement, les phases de propagation de la levure et de fermentation alcoolique, en détaillant les différents substrats susceptibles d'être générés par le procédé, substrats utilisables pour les étapes de propagation de levure et production d'éthanol.
   La composition en sucres des cinq substrats est la suivante :
   (1) Cellulose (polymère de glucose) et hémicellulose solubilisée (monomères et oligomères d'hexoses et pentoses). Substrat solide.
   (2) Cellulose et hémicellulose hydrolysées (monomères et oligomères d'hexoses et pentoses). Substrat liquide contenant des solides en suspension.
   (3) Cellulose (polymère de glucose). Substrat solide.
   (4) Hémicellulose solubilisée (monomères et oligomères d'hexoses et pentoses). Substrat liquide.
   (5) Cellulose hydrolysée (glucose). Substrat liquide contenant des solides en suspension.
La Figure 2 illustre l'évolution des concentrations en levures (en cellules/mL), en substrats (sucres C5 et C6) et en éthanol (en g/kg) au cours de la propagation sur hydrolysat lignocellulosique (ou SHF pour Separated Hydrolysis and Fermentation).
La Figure 3 illustre l'évolution des concentrations en levures (en cellules/mL), en substrats (sucres C5 et C6) et en éthanol (en g/kg) au cours de la propagation sur jus de C5.
La Figure 4 illustre l'évolution des concentrations en levures (en cellules/mL), en substrats (sucres C5 et C6) et en éthanol (en g/kg) au cours de la propagation SSP (propagation et saccharification simultanées) selon l'invention, à 10% MS et 10 mg protéines enzymatiques/g MS.
La Figure 5 illustre l'évolution des concentrations en levures (en cellules/mL), en substrats (sucres C5 et C6) et en éthanol (en g/kg) au cours de la propagation SSP selon l'invention, à 12% MS et 10 mg protéines enzymatiques/g MS.
La Figure 6 illustre l'évolution des concentrations en levures (en cellules/mL), en substrats (sucres C5 et C6) et en éthanol (en g/kg) au cours de la propagation SSP selon l'invention, à 10% MS et 7 mg protéines enzymatiques/g MS.
La Figure 7 illustre l'évolution des concentrations en levures (en cellules/mL), en substrats (sucres C5 et C6) et en éthanol (en g/kg) au cours de la propagation SSP selon l'invention, à32°C.
La Figure 8 compare l'évolution des concentrations en levures (en cellules/mL), en substrats (sucres C5 et C6) et en éthanol (en g/kg) au cours de la propagation SSP selon l'invention, à 30 et 32°C.
La Figure 9 illustre l'évolution des concentrations en levures (en cellules/mL), en substrats (sucres C5 et C6) et en éthanol (en g/kg) au cours de la propagation SSP selon l'invention, à 10% MS et 7 mg protéines enzymatiques/g MS, avec et sans addition d'acétate.
La Figure 10 illustre l'évolution des concentrations en levures (en cellules/mL), en substrats (sucres C5 et C6) et en éthanol (en g/kg) au cours de la propagation SSP selon l'invention, à 10% MS et 10 mg protéines enzymatiques/g MS, avec et sans addition d'acétate.
La Figure 11 compare l'évolution des concentrations en levures (en cellules/mL) au cours de la propagation sur hydrolysat lignocellulosique (SHF) et de la propagation SSP à 10% MS et 7 mg protéines enzymatiques/g MS avec et sans addition d'acétate.
La Figure 12 compare l'évolution des concentrations en levures (en cellules/mL) au cours de la propagation sur jus de C5 et de la propagation SSP à 10% MS et 7 mg protéines enzymatiques/g MS avec et sans addition d'acétate.
La Figure 13 illustre les concentrations en sucres (C5 et C6) et en éthanol (en g/kg) au cours de la fermentation SSCF sur paille de blé avec addition d'acétate (qsp 4 g/kg) ensemencée avec la levure I-4783 propagée en SSP.

Selon la Figure 1, la biomasse végétale prétraitée peut être utilisée comme substrat en propagation de levure et/ou en fermentation alcoolique sous cinq formes différentes :
- La solution la plus répandue consiste à réaliser une hydrolyse du marc complet en présence de cellulases, afin d'obtenir un hydrolysat lignocellulosique liquide (2) comprenant un mélange de monomères d'hexoses (C6) et de pentoses (C5), ainsi que des oligomères en faible quantité.
- Un autre substrat courant est l'hydrolysat hémicellulosique également appelé « jus de C5 » (4), qui correspond à la fraction soluble du substrat en sortie de prétraitement.
- Cette extraction de l'hydrolysat hémicellulosique (4) en sortie de prétraitement aboutit à du marc cellulosique riche en cellulose (3) qui peut être hydrolysé grâce à des cellulases de la même façon que le marc complet. Dans ce cas, l'hydrolysat cellulosique obtenu contient majoritairement des monomères de glucose (5).
- Ce marc cellulosique riche en cellulose (3), qui se présente sous forme solide, peut également être utilisé sans hydrolyse préalable. Dans ce cas, les cellulases sont ajoutées en fermentation et l'hydrolyse de la cellulose et la consommation de glucose sont réalisées simultanément.
- Enfin, l'intégration du procédé la plus poussée consiste à utiliser le marc prétraité brut (1) comme substrat de fermentation sans étape intermédiaire d'hydrolyse ni de séparation. Dans ce cas, les cellulases sont ajoutées en fermentation et l'hydrolyse de la cellulose est réalisée simultanément à la consommation du xylose et des hexoses. Cette dernière option pour l'étape de fermentation alcoolique est connue sous le nom de saccharification et co-fermentation du xylose et des hexoses simultanée (Simultaneous Saccharification and Co-Fermentation ou SSCF).

La propagation des levures qui seront utilisées pour l'étape de fermentation relève de problématiques différentes, notamment en termes de transfert d'oxygène.

Elle est classiquement effectuée sur jus de C5 (4). Ainsi la demande de brevet US2014/0065700 mentionne une propagation effectuée sur jus de C5 et la demande WO2009/155633 indique comme essentielle l'étape préalable d'hydrolyse de la cellulose.

La propagation est peu traitée dans la littérature scientifique, et la propagation réalisée simultanément à l'hydrolyse n'a jamais été décrite. Le substrat utilisé est soit la fraction soluble en sortie de prétraitement (ou jus de C5), soit un hydrolysat lignocellulosique obtenu par hydrolyse préalable de la totalité du végétal prétraité. Après propagation, les levures sont utilisées soit pour fermentation (avec (SSF) ou sans saccharification), soit pour produire des protéines d'intérêt (Duarte et al., 2008 ; Applied Biochem Biotechnol, 148 : 119-29 ; Meyer et al., 1992 ; Biotechnol Bioeng, 40 (3) : 353-8 ; Holder et al., 1989 ; Biological Wastes, 28 (4) : 239-46 ; Gonzalez-Valdes & Moo-Young, 1981 ; Biotechnology Letters, 3 (3) : 143-8). Bellissimi & Richards (Bellissimi E, Richards C: Yeast propagation. Dans The alcohol textbook, a reference for the beverage, fuel and industrial alcohol industries, 5th edition, Edited by Ingledew WM, Kelsall DR, Austin GD, Kluhspies C. Nottingham : University Press; 2009:145-159) indiquent que la méthode de production des levures industrielles est une propagation aérobie, dans laquelle il n'y a pas de production d'alcool et un taux maximal de **cellules** est atteint. En effet, pour la levure, la capacité de croissance pendant une longue période et dans des conditions strictement anaérobies est limitée.

L'un des inconvénients du marc prétraité brut avant liquéfaction ou hydrolyse, est sa viscosité. Pour cette raison, l'étape de liquéfaction/solubilisation ou d'hydrolyse est présumée essentielle. Aucun document ne décrit ni ne suggère un procédé de saccharification et propagation simultanées.

La demande de brevet WO2011/56991 A1 décrit un procédé de saccharification et fermentation simultanées, avec éventuellement une propagation aérée, en parallèle, dans le milieu liquéfié riche en hexoses qui servira à la fermentation. La demande de brevet WO 2010/014817 A2 décrit un procédé visant à améliorer la qualité et/ou la quantité de l'organisme fermenteur (levure) pendant la phase de fermentation. La demande de brevet WO2014/72232 A1 décrit un procédé de propagation aérobie dans un hydrolysat lignocellulosique (utilisé comme source de carbone), dans lequel l'hydrolysat est ajouté en mode « fed-batch » de manière à obtenir et à maintenir un pH donné dans le réacteur. La demande de brevet US2014/0273167 A1 décrit un procédé aérobie de propagation de levures, sous agitation et aération, sur un substrat riche en hexoses résultant d'une hydrolyse. La demande de brevet US2014/0273166 A1 décrit un procédé de propagation de levures sur un substrat issu de la transformation d'une biomasse végétale, substrat préférentiellement riche en pentoses. Les levures soumises à propagation, dans ce cas d'espèce, sont des levures transformées, aptes à métaboliser les pentoses.

Le procédé de propagation sur jus de C5 nécessite une étape complexe d'extraction de la fraction liquide du marc prétraité. Le procédé de propagation sur hydrolysat riche en C6 (ou comprenant un mélange C5-C6) nécessite également une étape spécifique d'hydrolyse. Une telle étape est coûteuse et consommatrice de temps. Ainsi, il reste une amélioration souhaitable du procédé vers une version plus intégrée, conservant des performances satisfaisantes en termes de rendement, de productivité, et de taux de multiplication.

La présente invention propose un procédé de saccharification et de propagation simultanées. A l'encontre de l'art antérieur sur le caractère essentiel d'une étape d'hydrolyse ou d'extraction préalable à l'obtention d'un substrat apte à la propagation des levures, la Demanderesse propose un procédé utilisant le marc prétraité brut comme substrat de propagation de levures, sans étapes préalables d'hydrolyse ni de séparation. Ledit procédé combine la saccharification du marc brut par les cellulases et la croissance des levures à partir du ou des sucres en C5 disponibles et du ou des sucres en C6 libérés par l'hydrolyse enzymatique.

Le procédé de saccharification et propagation simultanées selon l'invention sera par la suite abrégé en SSP (selon son appellation anglo-saxonne Simultaneous Saccharification and Propagation).

Un avantage de la propagation selon l'invention est la réduction de temps et de coût grâce à l'intégration du procédé par suppression d'une étape.

Un autre avantage de l'invention est la limitation en sucres fermentescibles due à l'hydrolyse enzymatique réalisée simultanément qui permet d'atteindre un rendement de production de biomasse élevé sans imposer un protocole de fed-batch (aussi appelé fermentation discontinue alimentée).

Un autre avantage de l'invention est que le taux de glucose continuellement bas favorise la consommation du xylose, habituellement inhibée en présence de glucose.

Un autre avantage de l'invention est que la forte teneur finale en biomasse (de l'ordre de 17,4 g/kg) permet de limiter la taille de l'unité de propagation de levure, ainsi que la dilution du moût de fermentation due à l'ensemencement qui ne représente que 3% du volume de fermentation SSCF.

Un autre avantage de l'invention est que l'impact des inhibiteurs présents dans le marc prétraité sur les performances de croissance est significativement réduit.

Enfin, un autre avantage de l'invention est que la consommation du glucose au fur et à mesure de sa libération par l'hydrolyse enzymatique limite les risques de contamination.

### Description détaillée de l'invention

Le procédé de saccharification et propagation simultanées selon l'invention s'applique sur une biomasse prétraitée. Ladite biomasse est un matériel lignocellulosique, autrement dit un matériel qui contient de la lignocellulose. Le matériel lignocellulosique peut contenir d'autres constituants comme du matériel cellulosique (cellulose, hémicellulose), ainsi que des sucres, fermentescibles ou non et des pectines. D'une manière générale, le matériel lignocellulosique est issu de matériel végétal : tiges, feuilles, coques, enveloppes de plantes, feuilles, ramures ou bois d'arbres. Le matériel lignocellulosique peut également être issu de matériel herbacé, de résidus d'agriculture, de résidus forestiers, de déchets solides municipaux ou d'effluents de papèterie.

La biomasse utilisée dans le procédé peut être issue de miscanthus, de peuplier, ou de paille de blé.

Le matériel lignocellulosique doit être prétraité pour casser la lignine et la structure cristalline de la cellulose. Ceci facilite la solubilisation de l'hémicellulose et de la cellulose et leur accessibilité pour les enzymes susceptibles d'être utilisées dans le traitement de la biomasse. Tout moyen de prétraitement, en particulier d'imprégnation puis de prétraitement, connu de l'homme du métier, peut convenir. Schématiquement, le prétraitement peut être chimique, mécanique ou biologique. Le prétraitement chimique comprend le traitement par un agent catalytique acide basique, notamment l'acide sulfurique, ou par des solvants organiques, le dioxyde de soufre ou le dioxyde de carbone. L'oxydation en milieu liquide et l'hydrothermolyse à pH contrôlé sont aussi considérées comme des traitements chimiques. Le prétraitement mécanique correspond à tout traitement mécanique ou physique tel le broyage, l'irradiation, l'explosion à haute pression ou à haute température (explosion à la vapeur). Selon certains modes de réalisation, les traitements chimique et mécanique peuvent être combinés, séquentiellement ou simultanément.

Selon un mode de réalisation avantageux, le prétraitement de la matière première comprend les étapes suivantes :
- imprégnation en présence d'un agent catalytique chimique acide ou basique, en particulier un catalyseur acide, préférentiellement l'acide sulfurique, dans des proportions comprises entre 0,1 et 2,0 % en poids, préférentiellement 0.5%. Avantageusement, ladite imprégnation est mise en oeuvre à une température comprise entre environ 50°C et environ 80°C, en particulier comprise entre environ 60°C et 70°C, préférentiellement à environ 65°C.
- injection de vapeur d'eau à une température comprise entre environ 120°C et environ 250°C, en particulier comprise entre environ 170°C et environ 190°C, préférentiellement à environ 180°C, à une pression comprise entre environ 5 et environ 15 bars, en particulier comprise entre environ 8 et environ 10 bars, préférentiellement à environ 9 bars, et pendant un temps compris entre 1 et 10 min, préférentiellement 5 min.

La biomasse végétale prétraitée peut alors être utilisée comme substrat pour la propagation des levures et/ou en fermentation alcoolique, comme l'indique la Figure 1 décrite *supra.*

La propagation est également appelée multiplication, prolifération ou production de biomasse. Le but est d'obtenir une quantité de biomasse optimale pour la fermentation. Le milieu de propagation issu de la biomasse prétraitée peut être riche en pentoses, riche en hexoses ou un mélange de pentoses et d'hexoses. Par pentoses, on entend des sucres présentant 5 atomes de carbone, également appelés sucres en C5 ou plus simplement C5. Les représentants monomériques principaux naturels des pentoses sont le D-xylose et le L-arabinose. Par analogie, les hexoses sont des sucres présentant 6 atomes de carbone, également appelés sucres en C6 ou plus simplement C6. Les représentants principaux des hexoses sous forme monomérique sont le glucose, le fructose, le mannose et le galactose.

La propagation SSP (saccharification et propagation simultanées) selon la présente invention s'adresse aux levures, aptes à transformer à la fois un ou plusieurs pentoses et un ou plusieurs hexoses.

L'expression « souche de levure » désigne une population homogène de cellules de levure. Une souche de levure est obtenue à partir de l'isolement d'un clone. Un clone donne naissance à une population de cellules obtenue à partir d'une seule cellule de levure.

Par l'expression « souche de levure dérivée », on désigne une souche de levure dérivée par un ou plusieurs croisements et/ou par mutation et/ou par transformation génétique.

Une souche de levure dérivée par croisement peut être obtenue par croisement interspécifique ou non. Une souche de levure dérivée par mutation peut être une souche de levure ayant subi au moins une mutation spontanée dans son génome ou au moins une mutation induite par mutagenèse. La ou les mutations d'une souche dérivée peuvent affecter le phénotype ou non. Par l'expression « mutagénèse », on désigne le processus d'apparition d'une mutation. Classiquement, deux méthodes sont possibles, la mutagénèse aléatoire et la mutagénèse insertionnelle ou dirigée. La première consiste en l'application d'un traitement physique (par exemple rayonnement UV) ou d'un traitement par des agents chimiques mutagènes, qui induiront de manière aléatoire des mutations dans le génome de l'organisme étudié. La seconde utilisera des méthodes de biologie moléculaire pour amener une modification précise (*i.e.* promoteur, gène, terminateur...), soit dans une région quelconque du génome, soit sur un locus précis. Par locus, on entend l'emplacement physique précis et invariable d'un gène sur un chromosome. Une souche de levure dérivée par transformation génétique est une souche de levure dans laquelle a été introduite une séquence d'ADN qui est de préférence apportée par un plasmide ou intégrée directement dans le génome.

Schématiquement, il est possible de distinguer quatre phases lors de la propagation d'une souche de levures : la phase dite « de latence » pendant laquelle aucune croissance n'est détectable et qui peut être assimilée à une période d'adaptation ; elle est suivie de la « phase de croissance » pendant laquelle les cellules se multiplient selon le taux maximal de croissance puis la « phase stationnaire » dans laquelle l'organisme fermentant entre lorsque la période de croissance maximale diminue puis cesse. Et enfin, la phase de déclin durant laquelle le nombre de cellules viables va diminuer. La propagation est d'une manière générale un processus aéré. L'aérobiose, ou aération du milieu de propagation, garantit un rendement de production de biomasse bien meilleur que l'anaérobiose. De même, des nutriments peuvent être apportés dans le milieu, tels qu'une source d'azote, une source de phosphore, des minéraux. Les vitamines et les composés organiques tels que les acides aminés ou les acides nucléiques sont rarement ajoutés en milieu industriel en raison de leur coût. Plus la phase de croissance sera rapide et courte, plus les contaminations microbiennes seront évitées. Une trop forte contamination de la propagation conduira à des pertes de rendement de production lors de l'étape ultérieure de fermentation. Pour limiter les contaminations, les antimicrobiens et les antibiotiques de type pénicilline ou virginiamycine, ou les extraits acides de houblon, peuvent être utilisés.

La propagation par SSP selon l'invention doit être effectuée en microaérobiose. Ceci signifie que le milieu est aéré mais la quantité d'oxygène fournie est limitante. La pression d'oxygène dissous est nulle, contrairement à l'aérobiose. L'oxygène dissous dans le milieu de fermentation est mesuré à l'aide d'une sonde à oxygène selon une méthode connue de l'homme du métier. La microaérobiose dans le procédé selon l'invention est obtenue par une aération et une agitation modérées. De façon préférentielle, l'aération est de 0,1 VVM (volume d'air/volume de milieu/minute, soit 60 mL pour un réacteur contenant 600 mL de milieu et par minute) et l'agitation est fixée autour de 500 rpm. Concrètement, l'agitation dépend de l'échelle à laquelle le procédé est mis en oeuvre ; en d'autres termes, l'homme du métier adapte en fonction du matériel, du volume dudit matériel, et de la dépense en énergie acceptable. Plus le volume de travail est élevé, plus l'agitation est faible.

La biomasse obtenue peut alors être investie dans un procédé de fermentation. La fermentation est conduite de préférence à 32°C, sous agitation modérée, par exemple 90 rpm. L'agitation est modérée pour ne pas être oxygénante. Le pH du milieu de fermentation est de préférence contrôlé, par exemple par le pouvoir tampon d'un couple acide/base. Le pH cible préféré dans le procédé selon l'invention est de 5,0. Lorsque la fermentation a pour but de produire de l'éthanol, la quantité d'éthanol présente dans le milieu de fermentation est mesurée par tout moyen approprié connu de l'homme du métier. Il peut s'agir d'une mesure directe de l'éthanol produit ou d'une mesure indirecte *via* un paramètre corrélé à la production d'éthanol, tel que la perte de masse. Par exemple, la production d'éthanol peut être mesurée par chromatographie, notamment par HPLC (High Performance Liquid Chromatography), par un kit enzymatique, ou par un dosage par le dichromate de potassium. La quantité de xylose et/ou de glucose présente dans le milieu est mesurée par tout moyen approprié connu de l'homme du métier, de préférence par chromatographie, notamment par HPLC.

L'homme du métier sait déterminer les conditions appropriées pour une fermentation alcoolique.

A titre d'exemple, on peut se référer aux conditions de fermentation alcoolique décrites dans le livre de référence « Yeast Technology », 2ème édition, 1991, G. Reed et T.W. Nagodawithana, publié par Van Nostrand Reinhold, ISBN 0-442-31892-8.

Le milieu de fermentation comprend les éléments suivants : au moins une source de carbone fermentescible, au moins une source d'azote, au moins une source de soufre, au moins une source de phosphore, au moins une source de vitamines et/ou au moins une source de minéraux.

La source de carbone est par exemple apportée sous la forme d'un sucre immédiatement assimilable par la levure, tel que le xylose, l'arabinose, le glucose, le fructose ou le galactose, un disaccharide de type saccharose et/ou un mélange de ces sucres.

Ces sucres peuvent être apportés sous forme de sirop, de mélasses, d'EP2 (Egout Pauvre issu de la 2ème cristallisation du sucre), d'hydrolysats de tout ou partie d'un matériel végétal et/ou d'un mélange de ceux-ci.

La source d'azote est par exemple apportée sous la forme d'extraits de levure, de sulfate d'ammonium, hydroxyde d'ammonium, di-ammonium phosphate, ammoniaque, urée et/ou une combinaison de ceux-ci.

La source de soufre est par exemple apportée sous la forme de sulfate d'ammonium, sulfate de magnésium, acide sulfurique et/ou une combinaison de ceux-ci.

La source de phosphore est par exemple apportée sous la forme d'acide phosphorique, phosphate de potassium, di-ammonium phosphate, mono-ammonium phosphate, et/ou une combinaison de ceux-ci.

La source de vitamines est par exemple apportée sous la forme de corn steep liquor, de mélasses, hydrolysat de levure, solution de vitamine pure ou d'un mélange de vitamines pures et/ou une combinaison de ceux-ci. La source de vitamines apporte à la levure l'ensemble des vitamines dans des quantités au moins équivalentes à celles recommandées dans les ouvrages de référence. Plusieurs sources de vitamines peuvent être associées.

La source de minéraux est par exemple apportée sous la forme de mélasses, un mélange de sels minéraux et/ou leur combinaison.

La source de minéraux apporte à la levure l'ensemble des macroéléments et oligoéléments dans des quantités au moins équivalentes à celles recommandées dans les ouvrages de référence. Plusieurs sources de minéraux peuvent être associées.

Une même substance peut apporter plusieurs éléments différents.

La propagation selon l'invention est caractérisée en ce que la saccharification et la propagation sont effectuées de façon simultanée.

Le marc prétraité brut est utilisé à raison d'une teneur en matière sèche comprise entre 8 et 15%, préférentiellement entre 10 et 12%, avantageusement à 10%. Selon un mode de réalisation de l'invention, le marc comprend environ 1/3 de matière sèche soluble (type « jus de C5) et 2/3 de matière sèche insoluble (type fibres lignocellulosiques).

Le marc prétraité brut est mis en contact avec une population de levures aptes à métaboliser les pentoses et les hexoses. Les levures sont ajoutées, préférentiellement sous forme sèche, à raison de 0,2 à 2 g/kg, autrement dit à raison de 0,2 à 2 g de matière sèche de levure par kilogramme de milieu complet préparé.

Le mélange marc prétraité brut et levures est additionné d'une combinaison de cellulases et d'hémicellulases qui permettent la saccharification. La saccharification correspond à l'hydrolyse des polysaccharides en sucres monomères solubles. Cela signifie que la concentration en sucres simples augmenterait dans le milieu s'ils n'étaient pas consommés par les levures pour la propagation, en parallèle de leur libération par les enzymes. Les cellulases permettent ainsi l'hydrolyse de la cellulose pour obtenir du glucose. Les exo-cellulases ou cellobiohydrolases agissent aux extrémités de la cellulose pour former le disaccharide cellobiose. Les endoglucanases agissent par clivage des liaisons internes de la cellulose en formant des oligosaccharides cellulosiques. Les cellobiases ou beta-glucosidases hydrolysent les oligopolymères cellulosiques et le cellobiose par leur extrémité réductrice en libérant du glucose.

Concrètement, on regroupe sous le terme cellulases un mélange de protéines enzymatiques. De façon préférentielle, les enzymes sont utilisées à raison de 5 à 15 mg de protéines (enzymes) par gramme de matière sèche. Avantageusement, elles sont utilisées à raison de 7 à 10 mg de protéines (enzymes) par gramme de matière sèche, préférentiellement 7 mg de protéines enzymatiques par gramme de matière sèche. Pour permettre une compréhension correcte et une comparaison entre les activités de différentes compositions présentant une activité de type cellulase, l'activité FPU (Filter Paper Unit) peut être utilisée comme référence. La commission biotechnologique de l'organisme international IUPAC (International Union of Pure and Applied Chemistry) recommande la procédure suivante: l'activité FPU est mesurée sur papier Whatman n°1 à la concentration initiale de 50 g.L⁻¹. Le but est de déterminer par dosage colorimétrique (à l'acide dinitrosalicylique, DNS) la quantité de sucres réduits issue du papier Whatman n°1. A titre d'exemple est déterminée la prise d'essai de la solution enzymatique à analyser qui libère l'équivalent de 2 g.L⁻¹ de glucose en 60 minutes. Les activités spécifiques sont obtenues en divisant les activités exprimées en IU.mL⁻¹ par la concentration en protéines ; elles sont exprimées en IU.mg⁻¹.

De façon avantageuse, la combinaison de cellulases et d'hémicellulases mises en œuvre dans un procédé selon l'invention correspondent à une composition enzymatique présentant une ou plusieurs activité(s) améliorée(s) par rapport à une composition contenant des protéines produites par le champignon natif. De telles cellulases sont connues de l'homme du métier, par exemple décrites par Durand et al., 1988 (Enzyme Microb. Technol., 10 : 341-346). Selon un mode préféré de réalisation de l'invention, les cellulases correspondent à une composition enzymatique telle que décrite dans la demande WO2010029259 A1, en particulier une composition enzymatique produite par des champignons filamenteux, préférentiellement *Trichoderma reesei.*

Le mélange est ensuite incubé, en micro-aérobiose, à une température comprise entre 25 et 38°C, préférentiellement entre 28 et 33°C, préférentiellement entre 30 et 32°C.

De façon avantageuse, le pH de la solution est autour de 5,0.

L'incubation est maintenue entre 24 et 50 heures, particulièrement entre 28 et 50 heures, plus préférentiellement entre 30 et 42 heures.

Avantageusement, la concentration cellulaire cible (en fin de propagation) est comprise entre 5,0 x 10⁸ et 1,0 x 10⁹ cellules par millilitre.

Les levures transformées aptes à métaboliser à la fois les pentoses et les hexoses peuvent être obtenues suivant des procédés décrits dans les demandes de brevets WO2010000464A1, WO2011128552A1 et WO2012072793A1. De façon avantageuse, lesdites souches sont également résistantes à l'acide acétique, obtenues selon un procédé tel que décrit dans la demande WO2013178915A1.

Selon un mode de réalisation de l'invention, la souche de levures utilisée métabolise préférentiellement le xylose et le glucose. En d'autres termes, selon un mode particulier de réalisation, l'invention porte sur un procédé de fermentation des sucres dérivés de biomasse lignocellulosique, préférentiellement des pentoses et des hexoses, utilisant un microorganisme fermentant, caractérisé en ce que ledit microorganisme a été produit directement sur marc prétraité brut, selon une méthode de saccharification et propagation simultanées.

Dans le contexte de l'invention, la souche de levure soumise à une propagation et saccharification simultanées selon l'invention est l'une des souches déposées à la CNCM (Collection Nationale de Cultures de Microorganismes, Institut Pasteur, 25 rue du Docteur Roux, 75724 Paris Cedex 15) le 24 mai 2012 sous les numéros I-4624, I-4625, I-4626, I-4627 ou à la souche déposée le 26 juin 2013, sous le numéro I-4783.

Selon un mode préféré de réalisation de l'invention, la propagation par SSP précède une étape de fermentation.

La présente invention a également pour objet un procédé de production d'au moins un produit de fermentation comprenant une étape de fermentation, en condition anaérobie ou semi-aérobie, par une levure propagée sur marc prétraité brut selon une méthode dans laquelle la saccharification et la propagation sont réalisées de façon simultanée.

Le produit de fermentation est notamment choisi parmi l'éthanol, un métabolite obtenu à partir de l'éthanol ou un métabolite secondaire.

Un produit de fermentation préféré selon l'invention est l'éthanol.

L'invention peut être mieux comprise à la lumière des exemples suivants qui ne sont en aucune façon limitatifs.

### Exemple 1 : Conditions de prétraitement et analyse de la composition du substrat

Le substrat utilisé pour ces essais est du marc brut de paille de blé issu d'un prétraitement selon la méthode suivante : la paille broyée est imprégnée dans de l'eau acide entre 0,1 et 2,0% en poids de H₂SO₄, puis prétraitée par explosion à la vapeur en continu à environ 50% de matière sèche pendant 1 à 10 min entre 170 et 190°C, préférentiellement à 180°C.

Ce marc brut de paille a été analysé par chromatographie liquide haute performance (CLHP ou HPLC) et les teneurs en sucres et inhibiteurs sont indiquées dans le Tableau 1.

**Tableau 1 : Composition du marc de paille utilisé pour la présente étude. Concentrations en g/kg.**

| | **Marc brut concentrations en g/kg** |
|---|---|
| **Teneur en MS (%)** | 46,02% |
| **Sucres** | |
| Cellulose | 170 |
| Cellobiose | 4 |
| Glucose | 9,7 |
| Xylose | 93,8 |
| Galactose | NQ |
| Arabinose | 10,9 |
| Mannose | 2,1 |
| **Composés inhibiteurs** | |
| Acide lactique | 0,7 |
| Acide acétique | 3,6 |
| Acide formique | 0,6 |
| 5-HMF | 0,4 |
| Furfural | 0,2 |

NQ signifie non quantifié autrement dit n'a pas été mesuré.

L'analyse des substrats a montré des teneurs en acide acétique et furfural classiques. La teneur en matières sèches du marc brut de paille est égale à 46,0%. Dans la plupart des essais de propagation réalisés par la suite, celui-ci a été utilisé à 10% de matière sèche, ce qui correspond à une dilution d'un facteur 4,6 des concentrations données ci-dessus lors de la mise en oeuvre dans le réacteur, alors que les deux autres substrats présentés dans le tableau, à savoir l'hydrolysat et le jus de C5, ont été utilisés sans dilution supplémentaire.

Les expériences suivantes ont été réalisées avec la levure *Saccharomyces cerevisiae* déposée à la CNCM (Collection Nationale de Cultures de Microorganismes, Institut Pasteur, 25 rue du Docteur Roux, 75724 Paris Cedex 15) le 26 juin 2013, sous le numéro I-4783. Les procédés mis en oeuvre sont décrits ci-après. A titre de comparaison, une autre souche de levure apte à métaboliser les pentoses et les hexoses donnerait des résultats similaires.

Les procédés de référence utilisent des substrats liquides, à savoir hydrolysat lignocellulosique, qui requiert une étape d'hydrolyse du marc prétraité préalablement à la propagation, ou jus de C5, (également appelé hydrolysat hémicellulosique), qui requiert une étape de séparation des sucres solubles du marc en sortie de prétraitement.

*A contrario,* le protocole de Saccharification et Propagation Simultanées (SSP) selon l'invention utilise le substrat solide de marc prétraité brut.

### Exemple 2 : Procédés de référence

### 2.1. Protocoles

Des antibactériens et des nutriments ont été ajoutés respectivement au jus de C5 et à l'hydrolysat lignocellulosique selon des quantités adaptées à la levure déposée à la CNCM sous le numéro I-4783 mise en oeuvre dans ces essais, à savoir :
NH₄OH 25%
Urée
H₃PO₄ 85%
Mélange de minéraux
Antibactérien

La composition du mélange de minéraux est présentée dans le Tableau 2. L'homme du métier saura adapter les proportions pour une efficacité optimale.

**Tableau 2 : composition du mélange de minéraux**

| **Composé** |
|---|
| MgSO₄.7H₂O |
| CuSO₄.2H₂O |
| MnCl₂.4H₂O |
| ZnSO₄.7H₂O |

Les réacteurs ont été inoculés à raison de 0,4 g/kg de levure sèche, et maintenus à une température de 30°C.

Le pH était maintenu à 5,0 par addition de KOH et H₂SO₄.

Pour les conditions de micro-aérobiose, le débit d'air était fixé à 0,1 VVM (volume d'air/volume de milieu/minute) et l'agitation était fixée à 500 rpm.

A titre indicatif, un débit d'air à 0,1 VVM est de 60 mL/min. pour un réacteur contenant 600 mL de milieu.

### 2.2. Résultats

### 2.2.1. Propagation sur hydrolysat lignocellulosique (SHF)

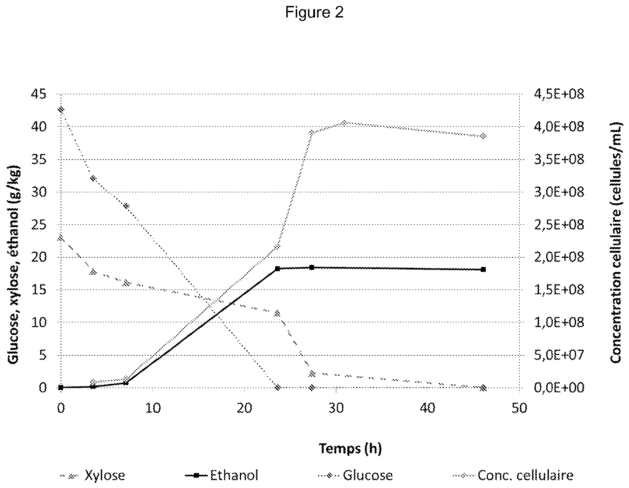

Les cinétiques de croissance des levures, de consommation de substrat et de production d'éthanol au cours de la propagation de levures en condition micro-aérobie (0,1 VVM) sur l'hydrolysat lignocellulosique sont présentées sur la Figure 2.

Cet essai de propagation a duré 46,1 h, mais la Figure 2 montre que la croissance des levures était terminée après environ 27 h de culture. La teneur finale en biomasse produite est estimée à 3,9.10⁸ cellules/mL. Le glucose a été consommé préférentiellement au xylose, comme c'est communément le cas en excès de glucose. 18 g/kg d'éthanol ont été produits. Remarque : La viabilité des levures au cours de la propagation n'est pas représentée sur la figure pour plus de lisibilité. Passé les premières heures de culture, elle est supérieure à 95% pour tous les essais présentés dans ces exemples.

### 2.2.2. Propagation sur jus de C5

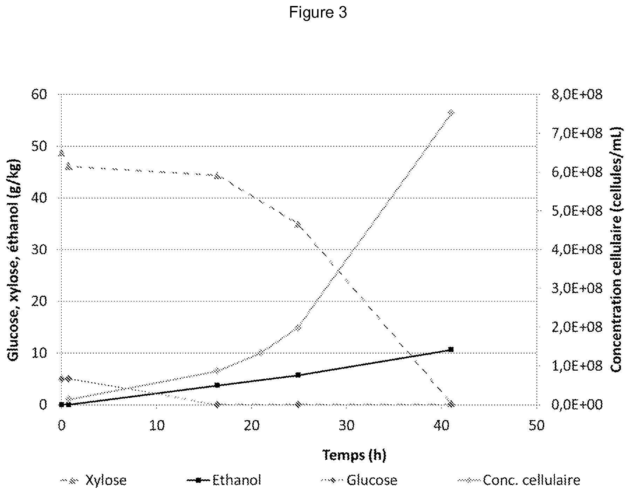

Les cinétiques de croissance des levures, de consommation de substrat et de production d'éthanol au cours de la propagation de levures en condition micro-aérobie (0,1 VVM) surjus de C5 sont présentées sur la Figure 3.

Cet essai de propagation sur jus de C5 montre une phase de latence plus longue (que celle de l'essai sur hydrolysat lignocellulosique), puis la biomasse croît rapidement pour atteindre 7,5.10⁸ cellules/mL après 41 h de culture. 10 g/kg d'éthanol ont été produits.

La différence de teneur finale en biomasse a déjà été observée. D'une manière générale, les rendements de production de levure sont plus élevés lors de la propagation sur jus de C5 que lors de la propagation sur hydrolysat lignocellulosique, majoritairement composé de sucre en C6 (glucose).

### Exemple 3 : Saccharification et propagation simultanées (SSP)

### 3.1. Procédé SSP selon l'invention

Le procédé de Saccharification et Propagation Simultanées est également mis en oeuvre dans un réacteur comprenant :
De l'eau, du marc prétraité brut cellulosique (substrat solide) mis en oeuvre à raison respectivement de 10% ou 12% de matière sèche (MS) dans la plupart des essais, et des nutriments tels qu'indiqués *supra* pour les procédés de référence.

La propagation était initiée par l'addition simultanée des cellulases (à raison respectivement de 7 et 10 mg de protéines par gramme de MS) et de la levure sèche à raison de 0,4 g/kg. Les enzymes utilisées dans les présents exemples peuvent être remplacées par des enzymes commerciales en quantité équivalente. A titre de comparaison, l'activité FPase (voir *supra* la mention des unités Filter Paper Unit) spécifique des cellulases utilisées dans les exemples est comprise entre 0,8 et 1,5 IU.mg⁻¹. Elles peuvent être remplacées par des enzymes du commerce dans les mêmes quantités (en IU.mg⁻¹).

La température était maintenue respectivement à 30°C ou 32°C.

Le pH était maintenu à 5,0 par addition de KOH et H₂SO₄.

Pour les conditions de micro-aérobiose, le débit d'air était fixé à 0,1 VVM (volume d'air/volume de milieu/minute) et l'agitation était fixée à 500 rpm.

Des échantillons ont été prélevés au cours des différents essais de propagation, afin de dénombrer les levures et de quantifier les sucres et les produits de fermentation par Chromatographie Liquide Haute Performance (CLHP).

Des hydrolyses enzymatiques totales ont été réalisées sur les échantillons finaux pour déterminer la teneur en cellulose non hydrolysée en fin de propagation.

### 3.2. Couples teneur en matière sèche (MS)-dose d'enzymes

Différents couples MS-dose d'enzymes ont été testés en SSP. Les pré-requis étaient : (i) une concentration en sucres fermentescibles qui doit permettre d'atteindre une teneur en biomasse de l'ordre de 15 g/kg en fin de propagation ; (ii) une cinétique d'hydrolyse de la cellulose qui doit limiter la quantité de glucose, de telle sorte (1) que le flux de carbone est orienté vers la production de biomasse plutôt que vers la production d'éthanol qui pourrait avoir lieu, même en présence d'oxygène, si la concentration en sucres est trop importante, et (2) que l'utilisation du xylose est favorisée sans que la productivité soit pénalisée ; et (3) la viscosité du mélange qui doit permettre l'agitation modérée et la micro-aération du milieu.

Les teneurs en MS et les doses d'enzymes testées sont indiquées dans le Tableau 3.

**Tableau 3 : teneurs en MS et doses d'enzymes testées en SSP**

| **Essai** | **Teneur en MS %** | **Dose d'enzymes mg prot./g MS** |
|---|---|---|
| 1 | 10 | 10 |
| 2 | 12 | 10 |
| 3 | 10 | 7 |

Rappel : comme pour les procédés de référence *(supra),* ces essais de propagation ont été ensemencés avec 0,4 g/kg de levure sèche, puis conduits à pH 5,0, à 30°C, avec une agitation modérée à 500 rpm et une micro-aération de 0,1 VVM.

### 3.2.1. Propagation en SSP à 10% de MS et 10 mg de protéines enzymatiques/g de MS

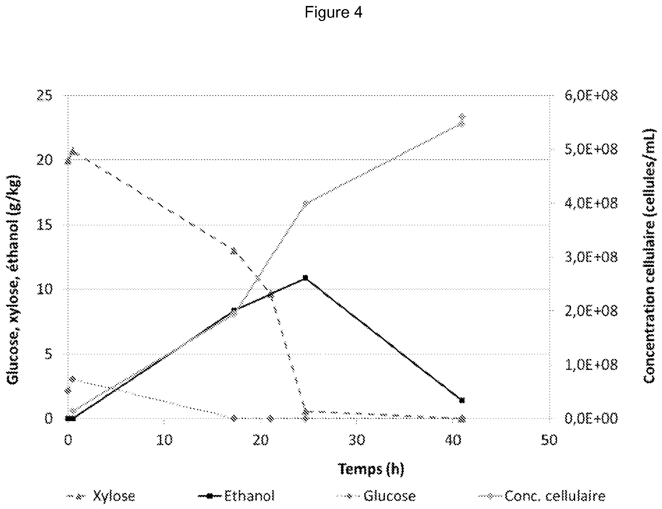

La levure I-4783 a été soumise à une propagation en condition micro-aérobie (0,1 VVM) sur le marc brut de paille (à 10% de MS), en présence de cellulases (10 mg protéines/g de MS) qui permettent de réaliser simultanément l'hydrolyse de la cellulose. Les cinétiques de croissance des levures et de production d'éthanol, ainsi que l'évolution des concentrations en glucose et xylose au cours de la propagation de levures sont représentées sur la Figure 4.

Résultat : cet essai de propagation a duré 41 h à l'issue desquelles 5,6.10⁸ cellules/mL ont été obtenues. 11 g/kg d'éthanol ont été produits, puis partiellement consommés au cours de cette propagation en SSP sur marc brut de paille. Par ailleurs, la teneur en MS employée a permis d'ajouter la totalité du substrat dans le pied de cuve initial tout en conservant une viscosité faible permettant l'agitation modérée et la micro-aération du milieu de culture. L'expérience a été répétée en prolongeant la croissance au-delà de 41h. Les résultats (non illustrés) étaient les suivants : 6,5.10⁸ cellules/mL ont été obtenues après 48h de culture et la cinétique de croissance était superposée à celle obtenue précédemment dans les mêmes conditions opératoires.

### 3.2.2. Propagation en SSP à 12% de MS et 10 mg de protéines enzymatiques/g de MS

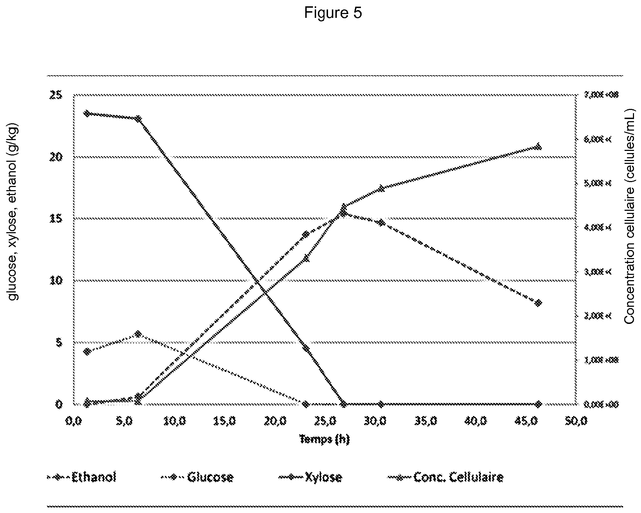

La levure I-4783 a été soumise à une propagation en condition micro-aérobie (0,1 VVM) sur le marc brut de paille (à 12% de MS), en présence de cellulases (10 mg protéines/g de MS) qui permettent de réaliser simultanément l'hydrolyse de la cellulose. Les cinétiques de croissance des levures et de production d'éthanol, ainsi que l'évolution des concentrations en glucose et xylose au cours de la propagation de levures sont représentées sur la Figure 5.

Résultat : cet essai de propagation a duré 46,3 h à l'issue desquelles 5,8.10⁸ cellules/mL ont été obtenues. 15 g/kg d'éthanol ont été produits, puis partiellement consommés au cours de cette propagation en SSP sur marc brut de paille.

L'augmentation de la teneur en MS de 10% à 12% n'a pas causé d'augmentation significative de la viscosité susceptible de perturber l'agitation modérée et la micro-aération du milieu de culture.

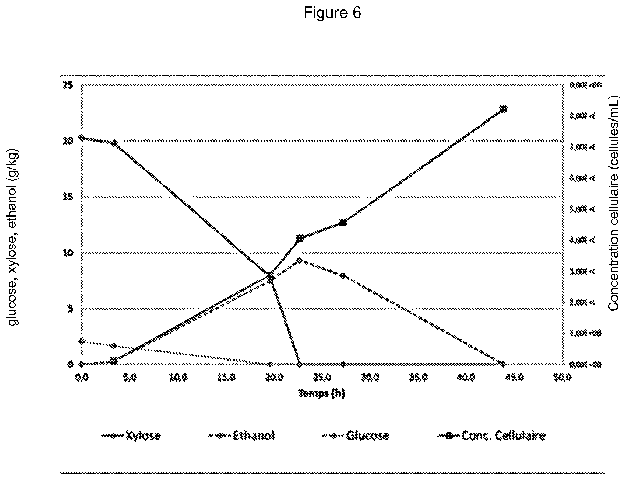

3.2.3. Propagation en SSP à 10% de MS et 7 mg de protéines enzymatiques/g de MS La levure I-4783 a été soumise à une propagation en condition micro-aérobie (0,1 VVM) sur le marc brut de paille (à 10% de MS), en présence de cellulases (7 mg protéines enzymatiques/g de MS) qui permettent de réaliser simultanément l'hydrolyse de la cellulose. Les cinétiques de croissance des levures et de production d'éthanol, ainsi que l'évolution des concentrations en glucose et xylose au cours de la propagation de levures sont représentées sur la Figure 6.

Résultat : cet essai de propagation a duré 43,9 h à l'issue desquelles 8,3.10⁸ cellules/mL ont été obtenues. 9,3 g/kg d'éthanol ont été produits, puis totalement consommés au cours de cette propagation en SSP sur marc brut de paille.

### Discussion

La comparaison des performances obtenues pour les essais de SSP réalisés à différentes teneurs en MS et doses d'enzymes montre que :
- L'augmentation de la teneur en MS de 10 à 12 % pour les essais réalisés avec 10 mg protéines /kg MS entraine une augmentation de la production d'éthanol mais n'a pas d'impact positif sur la croissance des levures.
- La diminution de la dose d'enzymes de 10 à 7 mg protéines enzymatiques/g MS pour les essais réalisés à 10% MS entraine une limitation plus forte en glucose qui se traduit par une consommation plus rapide du xylose et un flux de carbone orienté davantage vers la production de biomasse. L'écart de rendement d'hydrolyse dû à la baisse de la dose d'enzyme est inférieur à 2% en fin de propagation.

### 3.3. Effet de la température

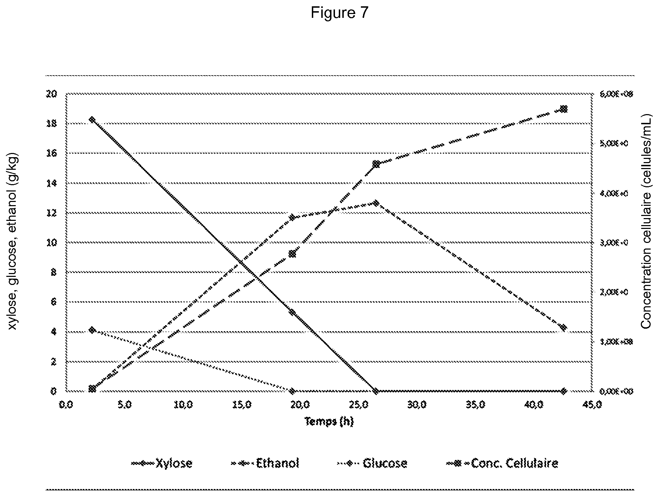

Dans le but d'observer l'effet de la température sur l'efficacité de la propagation en SSP, la levure I-4783 a été propagée à 32°C en condition micro-aérobie (0,1 VVM) sur le marc brut de paille (à raison de 10% de MS) en présence de cellulases (à raison de 10 mg protéines/g de MS). Les cinétiques de croissance des levures et de production d'éthanol, ainsi que l'évolution des concentrations en glucose et xylose au cours cet essai de propagation sont représentées sur la Figure 7.

Résultat : cet essai de propagation a duré 42,6h. Un ralentissement de la croissance a été observé en fin de culture. 12 g/kg d'éthanol ont été produits, puis partiellement consommés. La biomasse finale a été estimée à 5,7.10⁸ cellules/mL.

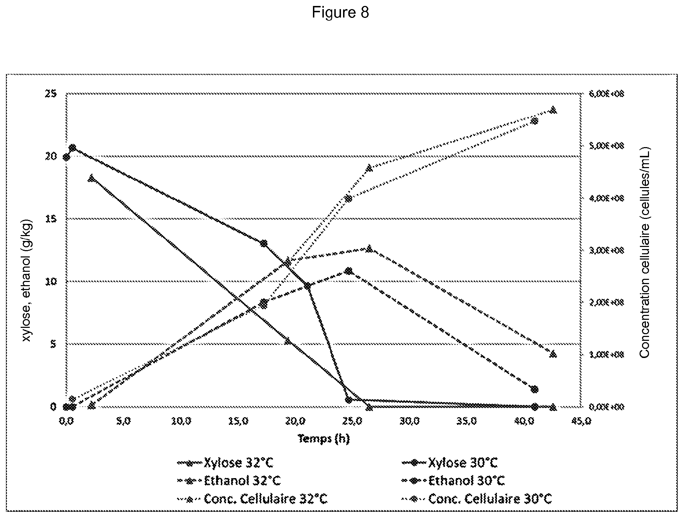

La Figure 8 compare l'évolution de la population de levures, ainsi que celle des concentrations en xylose et en éthanol, pour les essais de SSP réalisés respectivement à 30°C et 32°C, à 10% de MS et 10 mg protéines/g MS.

Aucun effet positif n'est observé sur la croissance des levures.

Il apparait que l'augmentation de température peut favoriser la prise du xylose, qui se traduit par une cinétique de production d'éthanol plus rapide.

L'augmentation de la température améliore l'hydrolyse enzymatique et augmente la quantité de sucres fermentescibles (de 12% dans ce cas). Si le moût de propagation est transféré intégralement pour ensemencer les fermentations alcooliques, les sucres résiduels, quelle que soit leur forme, représentent une faible proportion et seront utilisés au cours de la fermentation alcoolique.

### 3.4. Test de robustesse du procédé : essais de SSP en présence d'acétate en concentration élevée

Dans le but d'évaluer la robustesse du procédé de SSP, des essais ont été réalisés avec ajout d'acétate dans le milieu (QSP (quantité suffisante pour) 3 g/kg) pour simuler une toxicité plus élevée du marc prétraité. Ces essais ont été réalisés à pH 5,0, 30°C, avec 10% de MS et des ratios enzyme/substrat égaux à 7 mg protéines/g de MS et 10 mg protéines/g de MS. L'augmentation de la teneur en acétate dans le milieu de culture de 0,7 g/kg à 3 g/kg correspond à une augmentation de la teneur en acide acétique du marc prétraité de 3,6 g/kg à 13,8 g/kg.

Cette dernière concentration laisse une marge importante à l'augmentation de la teneur en composés volatils des substrats prétraités lors du passage à l'échelle industrielle.

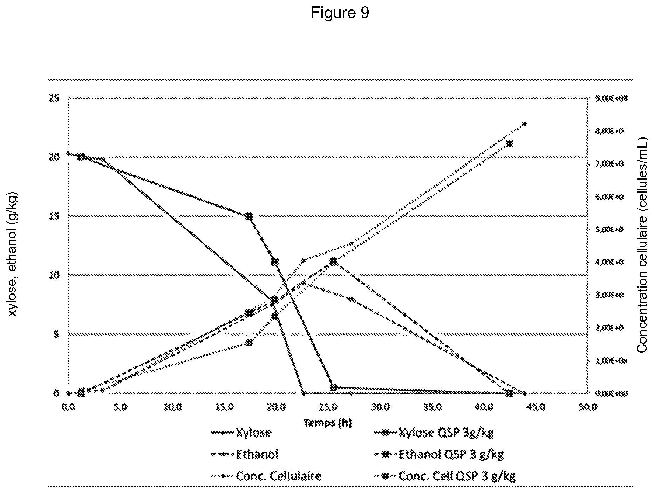

La cinétique de croissance des levures, ainsi que l'évolution des concentrations en xylose et éthanol au cours des essais de propagation réalisés avec et sans ajout d'acétate à 10% de MS et 7 mg protéines/g de MS sont illustrés sur la Figure 9.

La comparaison des cinétiques de SSP réalisées respectivement en présence de 0,7 g/kg ou 3,0 g/kg d'acide acétique montre que l'augmentation de la concentration en acétate ralentit l'utilisation du xylose et engendre un retard de croissance visible jusqu'à 25 h de culture. Le flux de carbone est légèrement dévié vers la production d'éthanol. Cependant, l'écart de concentration en biomasse disparait en fin de culture : 8,0.10⁸ cellules/mL ont été obtenues en 42,5 h pour l'essai à 3,0 g/kg d'acétate alors que 8,3.10⁸ cellules/mL ont été obtenues en 43,9 h pour l'essai à 0,7 g/kg d'acétate.

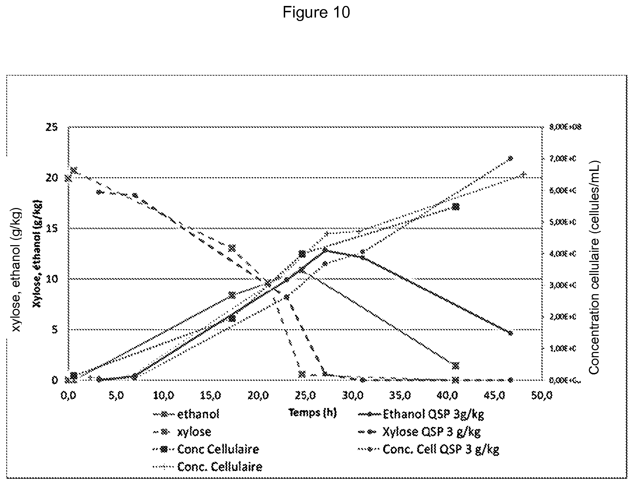

Les cinétiques de croissance des levures, ainsi que l'évolution des concentrations en xylose et éthanol au cours des essais de propagation réalisés avec et sans ajout d'acétate à 10% de MS et 10 mg protéines/g de MS sont illustrés sur la Figure 10.

La comparaison des cinétiques de SSP réalisées respectivement en présence de 0,7 g/kg ou 3,0 g/kg d'acide acétique montre que l'augmentation de la concentration en acétate engendre un retard de croissance visible jusqu'à 30 h de culture et que le flux de carbone est légèrement dévié vers la production d'éthanol. En fin de propagation, la teneur en biomasse produite en présence de 3 g/kg d'acétate dépasse la référence : 6,9.10⁸ cellules/mL ont été obtenues en 46,7 h contre 6,5.10⁸ cellules/mL en 48,1 h pour l'essai sans ajout d'acétate. Ces résultats montrent que l'augmentation importante de la quantité d'acide acétique dans le marc de paille prétraitée ne dégrade pas significativement les performances du procédé de propagation en SSP. Une telle robustesse ne peut-être attendue du procédé de propagation de levure sur jus de C5, la fermentation du xylose étant affectée beaucoup plus fortement que la fermentation du glucose par la toxicité du milieu de culture.

Là encore, deux ratios enzymes/substrats ont été testés. Les résultats sont cohérents avec ceux obtenus précédemment (*supra*), à savoir que la diminution de la dose d'enzymes améliore les performances du procédé de SSP.

### Exemple 4 : Comparaison des performances de croissance de la SSP selon l'invention avec celles obtenues avec les procédés de référence

L'évolution de la population de levures est comparée à celle obtenue pour les procédés de référence, dans des conditions de température, de pH, de micro-aération, d'agitation modérée et taux d'ensemencement identiques. Les quantités de sucres fermentescibles sont du même ordre de grandeur.

### 4.1. Comparaison avec la propagation sur l'hydrolysat lignocellulosique (SHF)

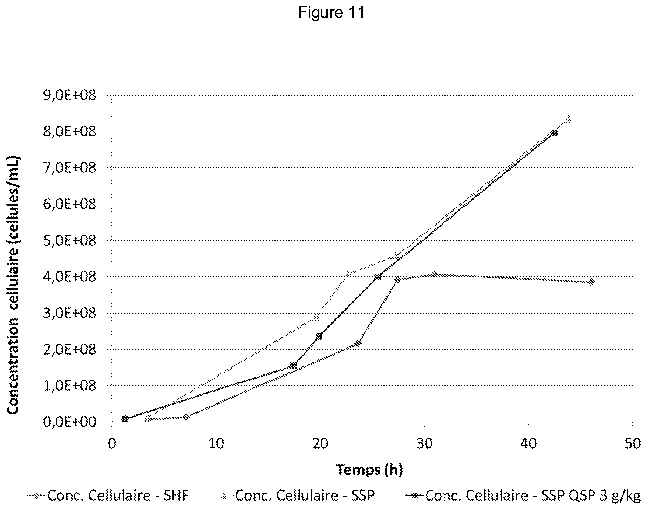

L'évolution de la population des levures au cours de la propagation sur hydrolysat lignocellulosique et des essais de SSP, avec et sans ajout d'acétate, à 10% de MS et 7 mg protéines/g de MS est représentée sur la Figure 11.

### Résultat et discussion

Les substrats respectifs de l'essai de propagation sur hydrolysat lignocellulosique et de l'essai de propagation SSP sans ajout d'acétate sont identiques, à une différence près : l'un a été préalablement hydrolysé. La croissance est plus lente sur l'hydrolysat, et ce dès le début de la propagation, ce qui peut s'expliquer par une teneur en acétate légèrement plus importante du fait de l'hydrolyse plus poussée du substrat en début de culture (1,0 g/kg vs 0,7 g/kg en SSP) ; la pression osmotique due aux sucres est également plus importante. En plus de sa meilleure cinétique, la propagation en SSP permet d'obtenir une quantité de biomasse largement supérieure (8,3.10⁸ cellules/mL vs. 3,9.10⁸ cellules/mL).

En outre, il est surprenant de constater que l'essai de propagation en SSP réalisé en présence de 3 g/kg d'acétate est également meilleur que l'essai de référence.

### 4.2. Comparaison avec la propagation sur jus de C5

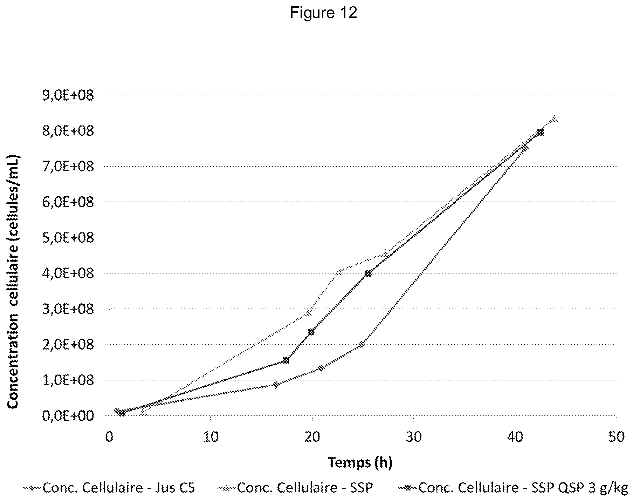

L'évolution de la population des levures au cours de la propagation sur jus de C5 et des essais de SSP, avec et sans ajout d'acétate, à 10% de MS et 7 mg protéines enzymatiques/g de MS est représentée sur la Figure 12.

### Résultat et discussion

La propagation réalisée sur jus de C5 a une cinétique de croissance significativement plus lente que les essais de propagation en SSP sur marc brut, cependant l'augmentation de la vitesse de croissance en fin de propagation lui permet d'atteindre une teneur finale en biomasse équivalente aux propagations SSP. Cependant, si la durée de propagation était réduite par rapport à l'essai présenté, l'avantage de la propagation SSP vis-à-vis de la propagation sur jus de C5 augmenterait.

Le jus de C5 utilisé pour cet essai de propagation était issu du même marc cellulosique de paille de blé que celui utilisé pour les essais de propagation en SSP. Il a été obtenu par mise en suspension du marc cellulosique dans de l'eau, puis séparation solide/liquide. Ce procédé d'obtention des jus de C5, extrait en réalité tous les éléments solubles du marc prétraité ; il peut donc être considéré et il a été vérifié que la teneur en inhibiteurs est proportionnelle à la concentration en xylose, ce qui fait du jus de C5 le substrat le plus concentré en inhibiteurs. Par ailleurs, le fait que la prise du xylose soit plus affectée par la toxicité du milieu que la consommation du glucose et que l'augmentation de la toxicité du marc prétraité entraine une augmentation plus rapide de la teneur en inhibiteurs dans le jus de C5 (car proportionnelle à la teneur en xylose) font que l'avantage du procédé SSP va augmenter avec la toxicité du marc prétraité. Ainsi, l'augmentation de la teneur en acétate de 0,7 g/kg à 3,0 g/kg dans le pied de cuve de la SSP, qui dégrade légèrement la cinétique de croissance en SSP, correspond à une augmentation de la teneur en acétate de 1,6 g/kg à environ 7 g/kg dans le jus de C5, ce qui pénalise fortement la croissance de la levure.

### 4.3. Synthèse et comparaison des performances de propagation

Le Tableau 4 fournit pour chaque essai de propagation réalisé :
- La concentration en sucres mise en oeuvre dans le milieu de culture (quantité totale et quantité fermentescible pour les essais réalisés en SSP),
- La concentration finale en biomasse obtenue (en cellules/mL),
- Le rendement de production de biomasse (rapporté à la teneur en sucres fermentescibles et rapporté à la teneur en MS),
- Une estimation du rendement de production de levure en g de levure/g de sucres fermentescibles.

**Tableau 4 : quantités de sucres mises en oeuvre, concentrations en biomasse obtenues et rendements de production de biomasse pour les différents essais de propagation (cell signifie cellules, masse ini. et masse fin. signifient respectivement masse initiale et masse finale).**

| **Conditions** | **Durée h** | **Potentiel sucre g/kg** | **Sucres fermentes. g/kg** | **Biomasse finale Cell/mL** | **Mas se ini. 9** | **Mas se fin. 9** | **Rendement Cell/g sucres fermentes.** | **Rendement g levure/g sucres** |
|---|---|---|---|---|---|---|---|---|
| Jus C5, 30°C | 41 | | 53,8 | 7,50E+08 | 600 | 607,4 | 1,41E+10 | 0,29 |
| SHF, 10% MS, 30°C | 46,1 | | 65,6 | 3,90E+08 | 600 | 593,7 | 5,88E+09 | 0,12 |
| 10% MS, 10 mg prot/g MS, 30°C | 41 | 64,5 | 53,3 | 5,60E+08 | 600 | 523,0 | 9,16E+09 | 0,19 |
| 10% MS, 10 mg prot/g MS, 30°C | 48,1 | 64,5 | 53,3 | 6,50E+08 | 600 | 557,0 | 1,13E+10 | 0,24 |
| 10% MS, 10 mg prot/g MS, 32°C | 42,6 | 64,5 | 59,9 | 5,70E+08 | 600 | 616,3 | 9,78E+09 | 0,20 |
| 10% MS, 7 mg prot/g MS, 30°C | 43,9 | 64,5 | 52,25 | 8,30E+08 | 600 | 604,9 | 1,60E+10 | 0,33 |
| 12% MS, 10 mg prot/g MS, 30°C | 46,3 | 77,3 | 66,0 | 5,80E+08 | 600 | 571,5 | 8,37E+09 | 0,17 |
| 10% MS, 7 mg prot/g MS, 30°C, 3g/kg d'acétate | 42,5 | 64,5 | 52,25 | 8,00E+08 | 600 | 599,3 | 1,53E+10 | 0,32 |
| 10% MS, 10 mg prot/g MS, 30°C, 3g/kg d'acétate | 46,7 | 64,5 | 58,7 | 6,90E+08 | 600 | 610,4 | 1,20E+10 | 0,25 |

### Remarques :

- Le potentiel sucre est calculé en faisant l'hypothèse d'un rendement d'hydrolyse de la cellulose égal à 100%.
- La quantité de sucres fermentescibles est calculée grâce à l'estimation de la quantité de cellulose résiduelle par hydrolyse enzymatique totale sur un échantillon pris en fin de culture.
- Pour l'estimation du rendement de production de biomasse en g de levure/g de sucres fermentescibles, la conversion est faite en considérant que 1 g de levure contient 4,8.10¹⁰ cellules (mesuré en fin de propagation sur jus de C5).
- La masse finale mesurée est anormalement faible pour les essais de propagation réalisés à 10% de MS avec 10 mg de protéines à 30°C, ce qui les pénalise lors du calcul des rendements.

### Résultats et Discussion

Le Tableau 4 montre que la concentration finale la plus importante en biomasse a été obtenue avec le procédé SSP mis en œuvre à 10% de MS et 7 mg de protéine/g de MS. La teneur finale en biomasse obtenue sur le jus de C5 est proche de celle obtenue avec le procédé de SSP dans les meilleures conditions, tandis que la propagation sur hydrolysat lignocellulosique aboutit à une teneur finale en biomasse significativement inférieure à tous les autres essais.

Le calcul du rendement de production de levure égale 1,6.10¹⁰ cellules/g de sucres fermentescibles pour l'essai le plus performant réalisé en SSP avec 10% de MS et 7 mg de protéine/g de MS à 30°C (et 1,5.10¹⁰ cellules/g de sucres fermentescibles pour l'essai réalisé avec une concentration accrue d'acide acétique). Le rendement de la propagation sur jus de C5 est légèrement inférieur : 1,4.10¹⁰ cellules/g de sucres fermentescibles ont été obtenues. Les autres conditions de SSP testées présentent des rendements de production de levure proches de 1,0.10¹⁰ cellules/g de sucres fermentescibles, tandis que l'essai de référence réalisé sur hydrolysat lignocellulosique présente un rendement de 5,9.10⁹ cellules/g de sucres fermentescibles, soit environ 3 fois moins que l'essai de SSP le plus performant.

Afin de comparer avec des références connues, le rendement de biomasse est estimé en g de levure/g de sucre fermentescible en considérant une conversion nombre de cellules/g de MS obtenues en fin de propagation alcoolique sur jus de C5. Selon des données connues de l'homme du métier, la propagation de référence sur hydrolysat lignocellulosique a un rendement de l'ordre de 0,12 g/g.

La meilleure condition de SSP a permis d'obtenir 0,33 g de levure/g de sucres fermentescibles. Le taux de multiplication de la levure en propagation est alors supérieur à 40 (concentration finale estimée 17,4 g/kg de levure).

En termes de productivité, le procédé de propagation en SSP est également le plus performant, en effet :
- La productivité volumique moyenne est estimée à 0,38 g de levure/kg de moût/h pour le procédé SSP, contre respectivement 0,37 g/kg/h et 0,17 g/kg/h pour les propagations sur jus de C5 et sur hydrolysat lignocellulosique.
- La productivité volumique moyenne sur les 30 premières heures de culture est estimée à 0,33 g de levure/kg de moût/h pour le procédé SSP, contre 0,26 g/kg/h pour les propagations sur jus de C5 et sur hydrolysat lignocellulosique.

### Exemple 5 : Validation du procédé de propagation SSP : performances de la levure propagée en fermentation SSCF

La présente invention est un maillon intermédiaire essentiel dans un processus global industriel de fermentation alcoolique. Le présent exemple a pour but de valider que la levure obtenue à l'issue d'une propagation selon l'invention est efficace en fermentation alcoolique sur substrat lignocellulosique.

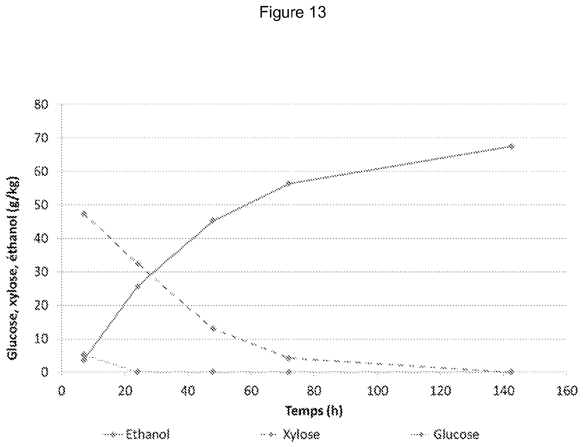

La levure I-4783 propagée en SSP a été utilisée pour ensemencer une fermentation SSCF (Simultaneous Saccharification and CoFermentation) ; les deux cultures ont été réalisées sur marc brut de paille. La SSCF a été conduite à 24% de MS avec 10 mg de protéine/g de MS, avec addition d'acétate dans le milieu (QSP 4 g/kg). Le réacteur a été ensemencé avec 2,4.10⁷ cellules/mL (soit environ 0,5 g/kg de levure) et le milieu a été maintenu à pH 5,5 et 33°C pendant 142,5 h. L'évolution des concentrations en glucose, xylose et éthanol au cours de cette fermentation est illustrée sur la Figure 13.

Cette fermentation SSCF présente une cinétique cohérente avec ce qui est obtenu habituellement. La levure consomme très rapidement le glucose libéré par les enzymes de sorte que la concentration en glucose est nulle dès les premières heures de fermentation. Le xylose libéré est majoritairement consommé en 72 h ; la cinétique de production d'éthanol est ensuite limitée par l'hydrolyse enzymatique. La teneur finale en éthanol est égale à 67,4 g/kg, ce qui correspond à un écart inférieur à 5% avec la concentration obtenue à l'issue des SSCF réalisées sans ajout d'acétate avec la levure I-4783 propagée sur jus de C5. Ce résultat permet de conclure que le procédé de propagation selon l'invention ne dégrade pas les performances de la levure produite par rapport à un procédé de propagation sur jus de C5 utilisé habituellement.

## Revendications

1. Procédé de propagation de levures, pour l'utilisation dans la production d'un produit de fermentation à partir de biomasse lignocellulosique, comprenant les étapes consistant à :
a. disposer d'un réacteur,
b. mettre en contact dans ledit réacteur :
- une population de levures aptes à métaboliser les pentoses et les hexoses, à raison de 0,2 à 2,0 g de matière sèche de levures par kg de milieu complet préparé, où les levures de la population sont des levures d'une souche de levure choisie parmi les souches déposées à la CNCM sous les numéros suivants : I-4624, I-4625, 1-4626, 1-4627 et I-4783,
- du marc brut issu du prétraitement de la biomasse lignocellulosique sans étapes d'hydrolyse ni de séparation, à raison d'une teneur en matière sèche (MS) comprise entre 8% et 15%,
- des nutriments, et
- des cellulases, à raison de 5 à 15 mg de protéine par gramme de MS,
c. incuber le mélange à une température comprise entre 25°C et 38°C, de préférence entre 28°C et 33°C, en microaérobiose,
dans lequel la saccharification du marc brut et la croissance des levures sont réalisées simultanément.

2. Procédé de propagation selon la revendication 1, dans lequel les pentoses sont le xylose et/ou l'arabinose.

3. Procédé de propagation selon la revendication 1 ou la revendication 2, dans lequel l'hexose est le glucose.

4. Procédé de propagation selon l'une quelconque des revendications 1 à 3, dans lequel l'incubation à l'étape c. est maintenue jusqu'à obtention d'une concentration cellulaire comprise entre 5,0 x 10⁸ et 1,0 x 10⁹ cellules par millilitre.

5. Procédé de propagation selon la revendication 1, dans lequel les levures sont ensemencées à raison de 0,3 à 0,6 g/kg sous forme de levures sèches, le marc brut est utilisé à raison de 10% de MS et les cellulases à raison de 7 mg de protéines par gramme de MS, le taux d'aération est fixé à 0,1 volume d'air/volume de milieu/minute (VVM), la température est fixée à 30°C et le pH du milieu est fixé à pH 5,0.

6. Procédé de production d'un produit de fermentation, à partir d'une biomasse lignocellulosique, comprenant séquentiellement les étapes de :
a. prétraitement de la biomasse lignocellulosique sans étapes d'hydrolyse ni de séparation pour obtenir un marc brut,
b. mise en contact d'une fraction du marc prétraité brut, à raison de 10% à 12% de matière sèche (MS), avec (i) une population de levures aptes à métaboliser les pentoses et les hexoses, où les levures de la population sont des levures d'une souche de levure choisie parmi les souches déposées à la CNCM sous les numéros suivants : 1-4624, I-4625, 1-4626, 1-4627 et I-4783, (ii) des cellulases à raison de 5 à 15 mg de protéine par gramme de MS, et (iii) de façon optionnelle, des nutriments,
c. incubation du mélange à une température comprise entre 25°C et 38°C, de préférence entre 28°C et 33°C, en microaérobiose, de façon à obtenir une saccharification et une propagation simultanées, jusqu'à l'obtention d'une concentration cellulaire comprise entre 5,0 x 10⁸ et 1,0 x 10⁹ cellules par millilitre,
d. transfert de tout ou partie des levures propagées pour mise en contact avec le moût de fermentation comprenant une source de pentoses et une source d'hexoses,
e. réalisation de la fermentation, en condition anaérobie ou semi-aérobie, et
f. obtention du produit de fermentation.

7. Procédé selon la revendication 6, dans lequel la biomasse lignocellulosique est une biomasse d'origine végétale.

8. Procédé selon la revendication 6 ou la revendication 7, dans lequel le produit de fermentation obtenu est de l'éthanol.

9. Procédé selon l'une quelconque des revendications 6 à 8, dans lequel les levures de la population mise en œuvre sont des levures de la souche déposée à la CNCM sous le numéro I-4783.

## Patentansprüche

1. Verfahren zur Vermehrung von Hefen zur Verwendung bei der Herstellung eines Fermentationsprodukts aus lignocellulosehaltiger Biomasse, umfassend die folgenden Schritte:
a. Bereitstellen eines Reaktors,
b. Inkontaktbringen in dem Reaktor:
- einer Population von Hefen, die Pentosen und Hexosen verstoffwechseln können, in einer Menge von 0,2 bis 2,0 g Trockenmasse der Hefen pro kg des vorbereiteten Vollmediums, wobei die Hefen der Population Hefen eines Hefestamms sind, der aus den bei der CNCM unter den folgenden Nummern hinterlegten Stämmen gewählt ist: 1-4624, I -4625, I -4626, I -4627 und I - 4783,
- rohem Trester aus der Vorbehandlung der lignocellulosehaltigen Biomasse ohne Hydrolyse- oder Trennungsschritte, in einer Menge von 8 % bis 15 % Trockenmasse (TM),
- Nährstoffen und
- Cellulasen in einer Menge von 5 bis 15 mg Protein pro Gramm TS,
c. Inkubieren der Mischung bei einer Temperatur zwischen 25 °C und 38 °C, bevorzugt zwischen 28 °C und 33 °C, in Mikroaerobiose,
wobei die Verzuckerung des Rohtresters und das Wachstum der Hefen gleichzeitig erfolgen.

2. Verfahren zur Vermehrung nach Anspruch 1, wobei die Pentosen Xylose und/oder Arabinose sind.

3. Verfahren zur Vermehrung nach Anspruch 1 oder Anspruch 2, wobei die Hexose Glucose ist.

4. Verfahren zur Vermehrung nach einem der Ansprüche 1 bis 3, wobei die Inkubation in Schritt c. bis zum Erreichen einer Zellkonzentration zwischen 5,0 x 10⁸ und 1,0 x 10⁹ Zellen pro Milliliter aufrechterhalten wird.

5. Verfahren zur Vermehrung nach Anspruch 1, wobei die Hefen in einer Menge von 0,3 bis 0,6 g/kg in Form von Trockenhefe ausgebracht werden, der Rohtrester in einer Menge von 10 % TS und die Cellulasen in einer Menge von 7 mg Protein pro Gramm TS verwendet werden, die Belüftungsrate auf 0,1 Volumen Luft/Volumen Medium/Minute (VVM) festgelegt wird, die Temperatur auf 30 °C festgelegt wird und der pH-Wert des Mediums auf pH 5,0 festgelegt wird.

6. Verfahren zur Herstellung eines Fermentationsprodukts aus lignocellulosehaltiger Biomasse, das nacheinander die folgenden Schritte umfasst:
a. Vorbehandlung der lignocellulosehaltigen Biomasse ohne Hydrolyse- oder Trennungsschritte, um einen Rohtrester zu erhalten,
b. Inkontaktbringen einer Fraktion des vorbehandelten Rohtresters in einer Menge von 10 % bis 12 % Trockensubstanz (TS) mit (i) einer Population von Hefen, die Pentosen und Hexosen verstoffwechseln können, wobei die Hefen der Population Hefen eines Hefestamms sind, der aus den bei der CNCM unter den folgenden Nummern hinterlegten Stämmen gewählt ist: I -4624, I -4625, I - 4626, I -4627 und I -4783, (ii) Cellulasen in einer Menge von 5 bis 15 mg Protein pro Gramm TS und (iii) optional Nährstoffe,
c. Inkubation der Mischung bei einer Temperatur zwischen 25 °C und 38 °C, bevorzugt zwischen 28 °C und 33 °C, unter mikroaeroben Bedingungen, so dass eine gleichzeitige Verzuckerung und Vermehrung bis zum Erreichen einer Zellkonzentration zwischen 5,0 x 10⁸ und 1,0 x 10⁹ Zellen pro Milliliter erreicht wird,
d. Übertragung der gesamten oder eines Teils der vermehrten Hefen zur Kontaktierung mit der Fermentationsmaische, die eine Pentosequelle und eine Hexosequelle enthält,
e. Durchführung der Fermentation unter anaeroben oder semi-aeroben Bedingungen und
f. Gewinnung des Fermentationsprodukts.

7. Verfahren nach Anspruch 6, wobei die lignocellulosehaltige Biomasse eine Biomasse pflanzlichen Ursprungs ist.

8. Verfahren nach Anspruch 6 oder Anspruch 7, wobei das erhaltene Fermentationsprodukt Ethanol ist.

9. Verfahren nach einem der Ansprüche 6 bis 8, wobei die Hefen der eingesetzten Population Hefen des bei der CNCM unter der Nummer I -4783 hinterlegten Stammes sind.

## Claims

1. A method for propagating yeast, for use in the production of a fermentation product from lignocellulosic biomass, comprising the steps of:
a. providing a reactor,
b. placing in contact in said reactor:
- a population of yeasts capable of metabolising pentoses and hexoses, with 0.2 to 2.0 g of yeast dry matter per kg of prepared complete medium, where the yeasts of the population are yeasts of a yeast strain selected from among the strains deposited with the CNCM under the following numbers: I-4624, I-4625, 1-4626, I-4627 and 1-4783,
- raw marc from the pre-treatment of the lignocellulosic biomass without any hydrolysis or separation steps, with a dry matter (DM) content between 8% and 15%,
- nutrients, and
- cellulases, with 5 to 15 mg protein per gram of DM,
c. incubating the mixture at a temperature between 25°C and 38°C, preferably between 28°C and 33°C, in microaerobiosis,
wherein the saccharification of the raw marc and the growth of the yeasts are carried out simultaneously.

2. The propagation method according to claim 1, wherein the pentoses are xylose and/or arabinose.

3. The propagation method according to claim 1 or claim 2, wherein the hexose is glucose.

4. The propagation method according to any one of claims 1 to 3, wherein the incubation in step c. is maintained until obtaining a cell concentration of between 5.0 x 10⁸ and 1.0 x 10⁹ cells per millilitre.

5. The propagation method according to claim 1, wherein the yeasts are seeded at a rate of 0.3 to 0.6 g/kg in the form of dry yeasts, the raw marc is used at a rate of 10% DM and the cellulases at a rate of 7 mg proteins per gramme of DM, the aeration rate is set at 0.1 air volume/medium volume/minute (VVM), the temperature is set at 30°C and the pH of the medium is set at pH 5.0.

6. A method for producing a fermentation product, from a lignocellulosic biomass, sequentially comprising the steps of:
a. pre-treating the lignocellulosic biomass without any hydrolysis or separation steps to obtain a raw marc,
b. placing in contact a fraction of the raw pre-treated marc, with 10% to 12% dry matter (DM), with (i) a population of yeasts capable of metabolising pentoses and hexoses, where the yeasts of the population are yeasts of a yeast strain selected from among the strains deposited with the CNCM under the following numbers: I-4624, 1-4625, 1-4626, 1-4627 and 1-4783,
(ii) cellulases with 5 to 15 mg of protein per gram of DM, and
(iii) optionally, nutrients,
c. incubating the mixture at a temperature between 25°C and 38°C, preferably between 28°C and 33°C, in microaerobiosis, so as to obtain simultaneous saccharification and propagation, until obtaining a cell concentration between 5.0 x 10⁸ and 1.0 x 10⁹ cells per millilitre,
d. transferring all or part of the propagated yeasts for contact with the fermentation wort comprising a source of pentoses and a source of hexoses,
e. carrying out the fermentation, under anaerobic or semi-aerobic conditions, and
f. obtaining the fermentation product.

7. The method according to claim 6, wherein the lignocellulosic biomass is a biomass of plant origin.

8. The method according to claim 6 or claim 7, wherein the obtained fermentation product is ethanol.

9. The method according to any one of claims 6 to 8, wherein the yeasts of the implemented population are yeasts of the strain deposited with the CNCM under the number 1-4783.
